Europäisches Patentamt

European Patent Office

Office européen des brevets

(11) Publication number: **0 110 044**
A1

## EUROPEAN PATENT APPLICATION

(21) Application number: 83109403.2

(22) Date of filing: 21.09.83

(51) Int. Cl.³: **C 12 N 15/00**, C 12 P 21/00, C 07 C 103/52, A 61 K 45/02, A 61 K 39/395

(30) Priority: 22.11.82 PC /JP82/004 45

(43) Date of publication of application: 13.06.84 Bulletin 84/24

(84) Designated Contracting States: AT BE CH DE FR GB IT LI LU NL SE

(71) Applicant: Takeda Chemical Industries, Ltd., 27, Doshomachi 2-chome Higashi-ku, Osaka-shi Osaka, 541 (JP)

(72) Inventor: Kikuchi, Masakazu, 4-16, Higashitokiwadai 7-chome Toyono-cho, Toyono-gun Osaka 563-01 (JP)
Inventor: Tsukamoto, Kyozo, A-204, 39 Senriyamanishi 4-chome, Suita-shi Osaka 565 (JP)
Inventor: Kurokawa, Tsutomu, 1-50, Suimeidai 1-chome, Kawanishi-shi Hyogo 666-01 (JP)

(74) Representative: von Kreisler, Alek, Dipl.-Chem. et al, Deichmannhaus am Hauptbahnhof, D-5000 Köln 1 (DE)

(54) Human immune interferon protein and production thereof.

(57) A substantially pure recombinant human immune interferon protein was obtained by growing a transformant having DNA containing a base sequence encoding human immune interferon to produce and accumulate human immune in interferon protein in the transformant cells, lysing the cells and purifying the resulting human immune interferon protein-containing solution with use of monoclonal antibody capable of binding to human immune interferon.

EP 0 110 044 A1

- 1 -

HUMAN IMMUNE INTERFERON PROTEIN AND PRODUTION THEREOF

## Field of the Invention

This invention relates to human immune interferon protein and a method of producing the same by the genetic engineering technique.

## Background Art

Interferons (hereinafter sometimes abbreviated to IFs) are proteins produced by higher animal cells on stimulation by viruses, nucleic acids, etc., and have antiviral and antitumor activities, among others.

Today, interferons are generally classified into three different types in characteristic properties, namely $\alpha$, $\beta$ and $\gamma$ types, and the $\alpha$ and $\beta$ types are induced by viruses or nucleic acids and the $\gamma$ type by mitogens etc.

Studies in type $\alpha$ IF (hereinafter abbreviated to IF-$\alpha$) and type $\beta$ IF (hereinafter abbreviated to IF-$\beta$) have considerably advanced and the mechanisms of production thereof have been elucidated to a considerable extent. Moreover, owing to advances in genetic engineering techniques, it is now possible to obtain IF-$\alpha$, IF-$\beta_1$ and IF-$\alpha_1$ as biologically active proteins in large amounts by cultivating Escherichia coli (IF-$\alpha$ and IF-$\beta_1$) and yeasts (IF-$\alpha_1$) (Goeddel, D. V. et al., Nature, 287, 411 (1980); Yelverton, E. et al., Nucleic Acids Res., 9, 731 (1981); Goeddel, D. V. et al., Nucleic Acids Res., 8, 4057 (1980); Hitzeman R. et al., Nature, 293, 717 (1981)). Furthermore, attempts at large-scale production are under way for the

0110044

purposes of clinical trials with them.

Type γ IF (hereinafter sometimes abbreviated to IF-γ) is produced from immunologically competent cells (immunocytes) under circumstances such that lymphocyte transformation (change to blast cells) or lymphokine production takes place, and accordingly it is also called immune interferon (hereinafter sometimes abbreviated to I-IF). I-IF is said to have higher anti-cell-proliferation or antitumor activity as compared with IF-α and IF-β, and therefore it is much expected of from the clinical application standpoint. However, due to various limitations, such as requirement of fresh lymphocytes for the production of I-IF, any efficient production systems have not been established yet. It is suggested that, in different experiment systems, different cell species can possibly produce different molecular species of I-IF. Their structures and properties still remain unknown in many aspects.

On the other hand, IFs are highly species-specific, and therefore IFs for use in humans must be human-derived. However, since large-scale production of human immune interferon is difficult, its clinical application is impossible at the present time, therefore development of a technology by which highly pure human I-IF can be produced easily in large amounts and at low costs has been waited for.

The present inventors have been engaged in research and development aiming at developing a technology for obtaining the desired human I-IF protein, which involves cloning of the human I-IF gene by utilizing the gene manipulation technique and introducing the thus-obtained recombinant DNA molecule into a host for the expression of the human I-IF gene. As a result, the inventors have completed the present invention.

Disclosure of the Invention

The present invention provides a substantially pure recombinant human immune interferon protein and a method of producing said protein which comprises growing a transformant having DNA containing a base sequence encoding human immune interferon and purifying the thus-obtained human immune interferon protein-containing liquid with use of monoclonal antibody capable of binding to human immune interferon.

Gray et al., believing that they could have cloned one of human I-IF genes, reported the amino acid sequence of a polypeptide deduced therefrom (Nature, 295, 503 (1982)). Then followed similar reports by Devos et al. (Nucleic Acids Research, 10, 2487 (1982)) and by Derynck et al. (ibid., 10, 3605 (1982)). So far, however, the presence of IF-like substances has been only presumed on the basis of the antiviral activity detected. No report mentions that a substantially pure human immune interferon protein could be obtained.

The present inventors have established a substantially pure human immune interferon and a method of producing the same and thus have completed the present invention.

The human I-IF-encoding DNA to be used in accordance with the present invention is, for example, a DNA containing a base sequence represented by the formula

(5') TGT TAC TGC CAG GAC CCA TAT GTA AAA GAA GCA
GAA AAC CTT AAG AAA TAT TTT AAT GCA GGT CAT TCA
GAT GTA GCG GAT AAT GGA ACT CTT TTC TTA GGC ATT
TTG AAG AAT TGG AAA GAG GAG AGT GAC AGA AAA ATA
ATG CAG AGC CAA ATT GTC TCC TTT TAC TTC AAA CTT
TTT AAA AAC TTT AAA GAT GAC CAG AGC ATC CAA AAG
AGT GTG GAG ACC ATC AAG GAA GAC ATG AAT GTC AAG
TTT TTC AAT AGC AAC AAA AAG AAA CGA GAT GAC TTC
GAA AAG CTG ACT AAT TAT TCG GTA ACT GAC TTG AAT
GTC CAA CGC AAA GCA ATA CAT GAA CTC ATC CAA GTG
ATG GCT GAA CTG TCG CCA GCA GCT AAA ACA GGG AAG
CGA AAA AGG AGT CAG ATG CTG TTT CGA GGT CGA AGA
GCA TCC CAG -X (3')                              (I)

wherein X is TAA, TGA or TAG.

The above-mentioned DNA (I) may have, at the 5'-end thereof,

5' ATG AAA TAT ACA AGT TAT ATC TTG GCT TTT CAG CTC TGC ATC GTT TTG GGT TCT CTT GGC 3'     (II) or ATG     (III).

The DNA (I) is preferably connected downstream from a promoter, such as tryptophan (trp) promoter, lactose (lac) promoter or protein chain elongation factor Tu (tufB) promoter, preferably trp promoter.

Referring to the formula (I), the oligonucleotide represented by X is preferably TAA.

In the present specification, drawings and claims, the symbols used are as defined in Table 1.

Table 1

DNA: Deoxyribonucleic acid

A: Adenine

T: Thymine

G: Guanine

C: Cytosine

RNA: Ribonucleic acid

dATP: Deoxyadenosine triphosphate

dTTP: Deoxythymidine triphosphate

dGTP: Deoxyguanosine triphosphate

dCTP: Deoxycytidine triphosphate

ATP: Adenosine triphosphate

EDTA: Ethylenediaminetetraacetic acid

SDS: Sodium dodecylsulfate

Gly: Glycine

Ala: Alanine

Val: Valine

Leu: Leucine

Ile: Isoleucine

Ser: Serine

Thr: Threonine

Cys: Cysteine

Met:     Methionine
Glu:     Glutamic acid
Asp:     Aspartic acid
Lys:     Lysine
Arg:     Arginine
His:     Histidine
Phe:     Phenylalanine
Tyr:     Tyrosine
Trp:     Tryptophan
Pro:     Proline
Asn:     Asparagine
Gln:     Glutamine
Z:       Carbobenzoxy
Boc:     t-Butoxycarbonyl
Mtr:     4-Methoxy-2,3,6-trimethylbenzenesulfonyl
Pme:     Pentamethylbenzenesulfonyl
OBu:     t-Butyl ester
ONB:     N-Hydroxy-5-norbornene-2,3-dicarboxiimido ester
DCC:     N,N'-Dicyclohexylcarbodiimide
DCU:     N,N'-Dicyclohexylurea
HONB:    N-Hydroxy-5-norbornene-2,3-dicarboxiimide
HOBt:    N-Hydroxybenzotriazole
CHA:     Cyclohexylamine
DCHA:    Dicyclohexylamine
TEA:     Triethylamine
TFA:     Trifluoroacetic acid
MSA:     Methanesulfonic acid
THF:     Tetrahydrofuran
DMF:     Dimethylformamide
MeOH:    Methanol
AcOEt:       Ethyl acetate

In accordance with the present invention, a double-stranded DNA coding for human I-IF can be produced, for example, by cultivating human I-IF-secreting cells, such as human peripheral blood lymphocytes, isolating a human I-IF-encoding messenger (m) RNA from the culture broth, synthesizing a single-stranded complementary DNA (cDNA) based thereon using, for instance, a reverse transcriptase, deriving a double-stranded DNA therefrom, inserting the same into a plasmid, transforming, for instance, Escherichia coli with the resulting plasmid, and isolating a cDNA-containing plasmid.

The human peripheral blood lymphocytes to be used here may be either unsensitized cells or sensitized cells.

The I-IF inducer to be used in the practice of the present invention may be of any kind provided that it can induce human lymphocyte cells to produce I-IF. Thus, there may be mentioned, among others, phytohemagglutinin (PHA), concanavalin A (ConA), staphylococcal enterotoxin A (SEA), neuraminidase-galactose oxidase (NAGO) and 12-0-tetradecanoyl-phorbol-13-acetate (TPA). These can be used either alone or in combination.

The I-IF induction can be effected by cultivating the cells in a per se known medium, such as RPMI-1640 medium or MEM medium, preferably RPMI-1640 medium containing fetal calf serum. The cultivation is conducted within the temperature range of 35-39°C, preferably 36-38°C, for a period of 12-72 hours, preferably 22-26 hours. The cells are collected by the per se known method and, following addition of an RNase inhibitor, such as guanidine thiocyanide, bentonite, heparin, polyvinylsulfuric acid, aurintricarboxylic acid, vanadium complex, diethyl pyrocarbonate (DPC) or sodium dodecylsulfate (SDS), the cells are lyzed by adding N-lauroylsarcosine, Tween 80 or Nonidet P-40, for instance. Then, RNA extraction is carried out. If necessary, after addition of phenol, phenol-chloroform or the like to this RNA-containing extract, the extraction procedure is repeated. The RNA fraction is collected by ethanol precipitation. Since it is known that most of mRNAs present in the cytoplasm of eukaryotic cells have a poly A sequence at the 3'-end thereof, poly(A$^+$) RNAs are collected using oligo(dT)cellulose or poly(U)sepharose, for instance, and fractionated by sucrose density gradient centrifugation. An aliquot of each fraction is injected into oocytes of Xenopus laevis for translation (Gurdon, J. B. et al., Nature, 233, 177 (1971)). The resulting protein is tested for antiviral activity. In this manner, a 12-16S fraction containing mRNA for I-IF can be obtained. The mRNA can also be purified by formamide-polyacrylamide gel electrophoresis or agarose gel electro-phoresis using glyoxal.

Using the thus-obtained mRNA as the template, a complementary DNA (cDNA) chain is synthesized, for example, by the per se known method using a reverse transcriptase, and the cDNA is converted to a double-stranded DNA (Maniatis, T. et al., Cell, 8, 163 (1976)).

This DNA is inserted, for instance, into the plasmid pBR322 at the PstI or SphI restriction endonuclease cleavage site, for example by the dG-dC or dA-dT homopolymer tailing method (Nelson, T. S., Methods in Enzymology, 68, 41 (1979), Academic Press Inc., New York). The recombinant DNA is introduced, for example, into Escherichia coli χ1776 for transformation. Transformant selection can be made based on the tetracycline resistance or ampicillin resistance. Separately, an oligonucleotide having a base sequence presumably corresponding to the amino acid sequence of I-IF polypeptide is chemically synthesized and then labelled with $^{32}$P to make a probe, and, using this, the previously obtained tetracycline- or ampicillin-resistant transformants are secondarily screened for the desired clones, for instance by the per se known colony hybridization method (Grunstein, M. and Hogness, D. S., Proc. Natl. Acad. Sci. USA, 72, 3961 (1975)).

The clones positive in this colony hybridization are examined for the base sequence, for example, by the Maxam-Gilbert method (Maxam, A. M. & Gilbert, W., Proc. Natl. Acad. Sci. USA, 74, 560 (1977)) or the dinucleotide synthesis chain termination method using phage M13 (Messing, J. et al., Nucleic Acids Res., 9, 309 (1981)) for confirmation of the presence of the I-IF gene. Then, the I-IF gene is cut out wholly or partly from the clone obtained in the above manner and connected downstream from an appropriate promoter and the SD (Shine and Dalgarno) sequence for introduction into an adequate host.

As examples of the promoter, the above-mentioned ones may be mentioned. Preferred hosts are Escherichia coli strains (294, W3110, C600, etc.), among which the strain 294 is especially preferred.

The strain 294 is a known organism (Backman, K. et al., Proc. Natl. Acad. Sci. USA, 73, 4174 (1976)) and also is deposited with the Institute for Fermentation, Osaka under the deposit number of IFO-14171.

The transformation of a host with the DNA according to the present invention is performed, for example, by the known method (Cohen, S. N. et al., Proc. Natl. Acad. Sci. USA, 69, 2110 (1972)).

The thus-obtained transformant is cultivated in a per se known medium.

The medium is, for example, M9 medium which contains glucose and casamino acid (Miller, J., Experiments in Molecular Genetics, 431-433 (Cold Spring Harbor Laboratory, New York, 1972)). For efficient promoter actuation, 3β-indolylacrylic acid or the like agent can be added as necessary.

The cultivation is generally carried out at 15-43°C for 3-24 hours. If necessary, aeration and/or stirring may be carried out.

After the cultivation, the cells are harvested by the known method and, for instance, following suspension in a buffer, lysed by means of ultrasonic treatment, lysozyme and/or freezing-thawing, for instance. Then, the supernatant is collected by centrifugation. For the cell lysis, freezing and thawing followed by lysozyme treatment is preferable.

Human I-IF in the above supernatant can be purified advantageously by using a monoclonal antibody capable of binding to human I-IF, preferably a monoclonal antibody against polypeptide of the formula:

(N)                                (C)
H-X-Lys Arg Ser Gln Met Leu Phe Arg Gly-Y-OH (IV) wherein X is a bond, or a peptide or amino acid residue having 1 to 16 amino acids counting from the C terminus of the peptide chain of

(N)
Ile Gln Val Met Ala Glu Leu Ser Pro Ala

                      (C)
Ala Lys Thr Gly Lys Arg and Y is a peptide or amino acid residue having 1 to 5 amino acids counting from the N terminus of the peptide chain of

(N)             (C)
Arg Arg Ala Ser Gln, which is novel polypeptide.

Referring to polypeptide (IV), it is preferable that Y is Arg Arg Ala Ser Gln or/and X is Lys Arg. Furthermore, it is preferable that polypeptide (IV) contains 15 to 25 amino acid residue.

Said monoclonal antibody can advantageously be used in the form of an antibody column.

The antibody column can be prepared, for instance, by coupling the above-mentioned monoclonal antibody in the purified state as obtained from the ascitic fluid inoculated with the hybridoma with an appropriate carrier, in the manner mentioned below.

The carrier may be of any kind provided that, after coupling, I-IF can be adsorbed specifically and efficiently and thereafter can be eluted by an adequate means. As an example, agarose gel in the form of beads, which is activated so as to facilitate the bonding of the primary amino group of a protein, for instance AFFI-GEL 10 (Bio-Rad Lab., USA) can be favorably used in the following manner. The reaction of AFFI-GEL 10 with the antibody is carried out in a buffer solution, such as 0.001-1 M, preferably 0.1 M, bicarbonate. Usable reaction conditions are 0°-20°C, 10 minutes to 24 hours, and varying pH, and preferred conditions are 4°C, 4 hours and pH 3-10. The quantitative ratio between AFFI-GEL 10 and the antibody may be selected within the range of up to about 50 mg of antibody per ml of AFFI-GEL, since, in this range, the amount of antibody coupled with AFFI-GEL increases as the amount of antibody increases. From the coupling efficiency viewpoint, the antibody is used in an amount of not more than 30 mg on said basis. The thus-produced antibody-carrier coupling product is washed well with the same buffer solution as used for the reaction and then allowed to stand for several days or treated with ethanolamine hydrochloride in a final concentration of 0.05 M at 4°C for an hour or by some other method so as to block the remaining unreacted active groups, and packed in an appropriate column to give an antibody column.

For the purpose of purification with the above antibody column, a human immune interferon protein-containing solution such as the supernatant by the cell lysis, for instance, is dissolved in an almost neutral buffer, such as a phosphate buffer or Tris hydrochloride buffer, and made adsorbed on the antibody column. The column is washed with the same buffer and then I-IF is eluted. Usable eluents are a weakly acidic solution (e.g. acetic acid solution), a polyethylene glycol-containing solution, a solution containing a peptide having a higher affinity for the antibody as compared with the objective protein, a high concentration salt solution, and a combination of these, among others. Those eluents which do not promote decomposition of human I-IF to a considerable extent are preferred.

The eluate from the column is neutralized with a buffer by the conventional method. If necessary, the purification procedure using the above antibody column can be repeated.

The thus-obtained human I-IF protein solution is dialyzed and, as necessary, can be made into a powder by lyophilization. In carrying out the lyophilization, a stabilizer, such as sorbitol, mannitol, dextrose, maltose or glycerol, can be added.

The thus-obtained human immune interferon protein, when assayed for antiviral activity in a test for evaluating the effect of inhibiting degeneration of human amnion-derived WISH cells by the vesicular stomatitis virus (VSV), shows a specific acitivity of not less than $10^7$ U/mg.

The IF activity in U/ml (units/ml) was determined in the following manner. An international standard IF-α for which the unit has been established and leukocyte-derived crude IF-γ were assayed in the test for estimating the inhibitory effect against cell degeneration caused in a human amnion-derived FL cell line by VSV, the titer of the lymphocyte-derived IF-γ was determined by comparison of the titers found, and said IF-γ was used as a laboratory standard IF-γ. In calculating the titer of IF-γ in a material, this laboratory standard IF-Y was always used in parallel in the above-mentioned assay in the WISH-VSV system and the titer calculation was performed based on the titer ratio.

The human immune interferon protein of the present invention preferably contains a polypeptide having the amino acid sequence of the formula

$(N)H-Y-Z_1$ Tyr $Z_2$ Gln Asp Pro Tyr Val Lys Glu
Ala Glu Asn Leu Lys Lys Tyr Phe Asn Ala Gly His
Ser Asp Val Ala Asp Asn Gly Thr Leu Phe Leu Gly
Ile Leu Lys Asn Trp Lys Glu Glu Ser Asp Arg Lys
Ile Met Gln Ser Gln Ile Val Ser Phe Tyr Phe Lys
Leu Phe Lys Asn Phe Lys Asp Asp Gln Ser Ile Gln
Lys Ser Val Glu Thr Ile Lys Glu Asp Met Asn Val

Lys Phe Phe Asn Ser Asn Lys Lys Lys Arg Asp Asp
Phe Glu Lys Leu Thr Asn Tyr Ser Val Thr Asp Leu
Asn Val Gln Arg Lys Ala Ile His Glu Leu Ile Gln
Val Met Ala Glu Leu Ser Pro Ala Ala Lys Thr Gly
Lys Arg Lys Arg Ser Gln Met Leu Phe Arg Gly Arg
Arg Ala Ser Gln-OH(C)                    (V)

wherein Y is Met or a chemical bonding, and $Z_1$ and $Z_2$ each is Cys or 1/2 Cys. The human I-IF protein of the present invention preferably contains said polypeptide in a purity of not less than 90%, in particular not less than 95%, on the dried basis.

A human immune interferon protein produced by the method of the present invention has the following characterization:

1)  It exhibits a molecular weight of 17,000±1,000 in SDS-polyacrylamide gel (17.5%) electrophoresis;

2)  It contains cysteine or half cystine or methionine as the amino-terminal amino acid;

3)  It combines with a monoclonal antibody against H-Lys-Arg-Lys-Arg-Ser-Gln-Met-Leu-Phe-Arg-Gly-Arg-Arg-Ala-Ser-Gln-OH.

The human interferon protein produced in accordance with the present invention can be used for the same purposes in the same manner as I-IF species obtained by the conventional methods. Owing to its smaller content in contaminant proteins and pyrogen, it can be used more safely as a substance for preparing injections, for instance.

The human I-IF protein of the present invention has antiviral, antitumor, cell proliferation-inhibiting and immunopotentiating activities. The human I-IF protein of the present invention can be used, for medicinal purposes, as it is or as a mixture with sterilized water, human serum albumin (HSA), physiological saline or/and other known physiologically acceptable carriers vehicle or diluent to produce a pharmacentical composition containing said human I-IF, and can be administered parenterally or locally. For instance, it can be administered intraveously or intramuscularly or by some other route in a dose of 100,000-100,000,000 units, preferably 50,000,000-60,000,000 units, per adult human per day.

The pharmaceutical preparations containing the human I-IF protein of the present invention may also contain other physiologically acceptable inert components such as salt, diluent, adjuvant, another carrier, buffer, binding agent, surfactant and preservative. For parenteral administration, the preparations are provided in the form of a suspension in a sterilized aqueous solution or a physiologically acceptable solvent in ampules or in the form of a sterilized powder (generally obtainable by lyophilization of an I-IF solution) to be diluted with a diluent prior to use.

Furthermore, the above-mentioned human immune interferon protein-containing preparations may further contain other active ingredients such as IF-α or IF-β or a lymphokine (e.g. interleukin 2) in an amount of 1-99% based on the substance of the present invention.

Brief Description of the Drawings

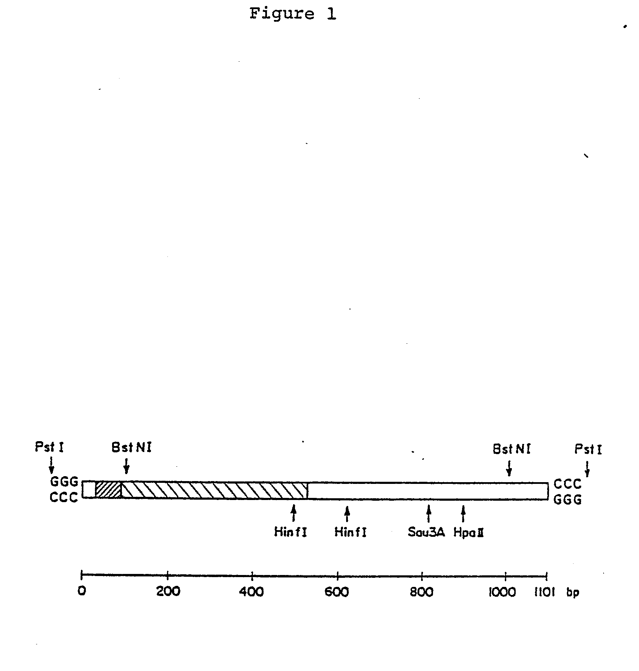

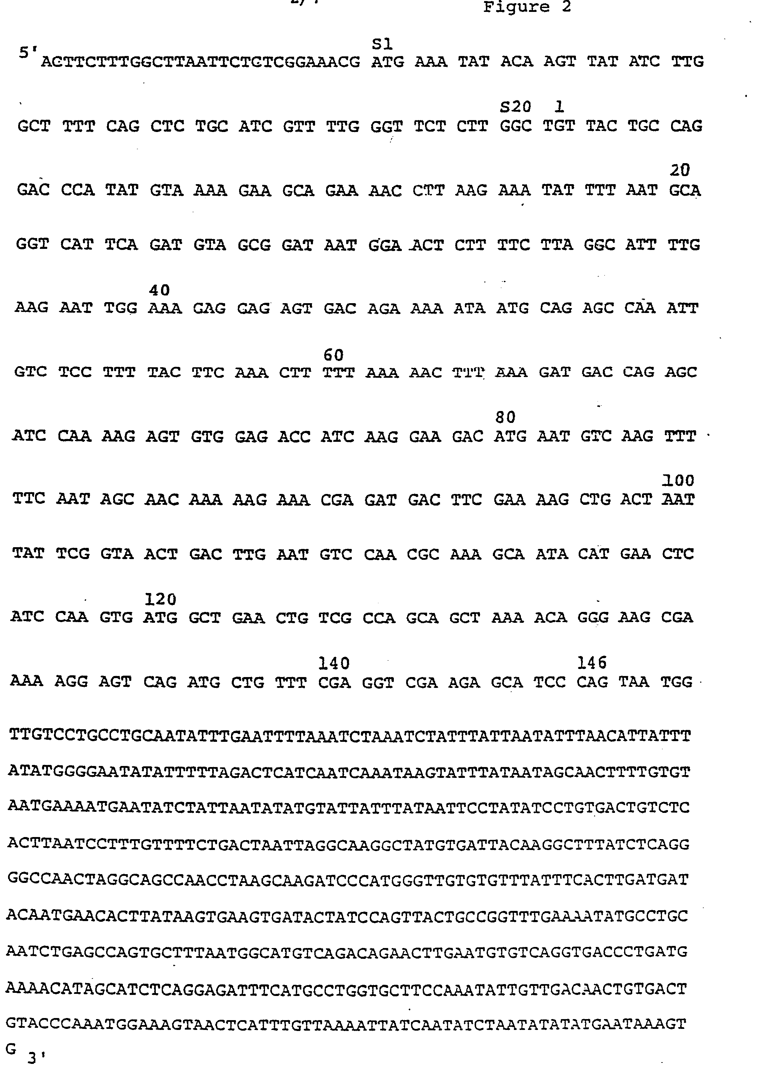

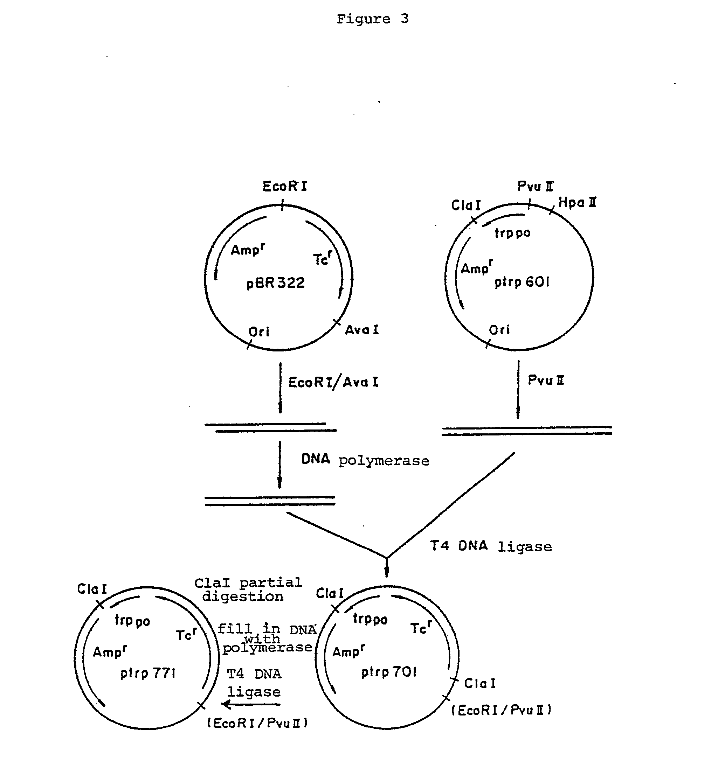

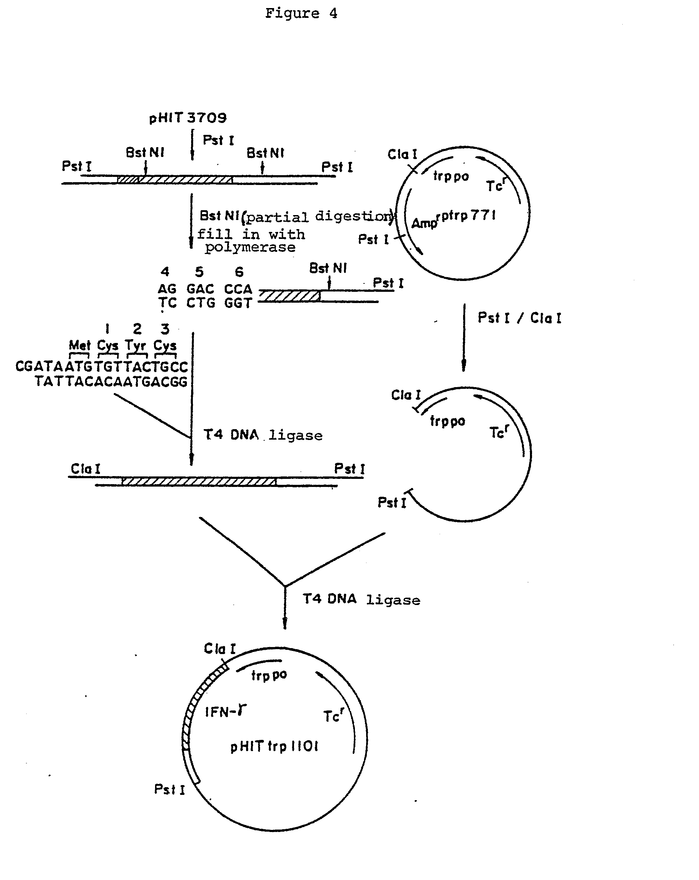

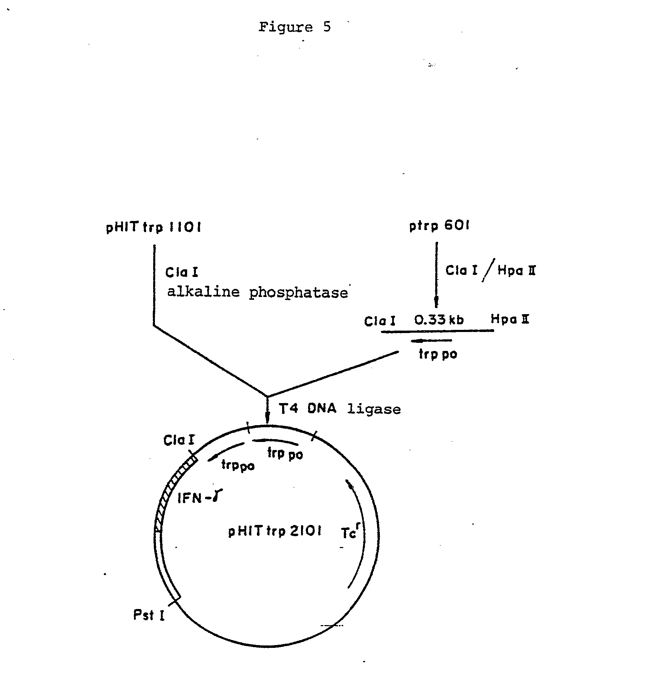

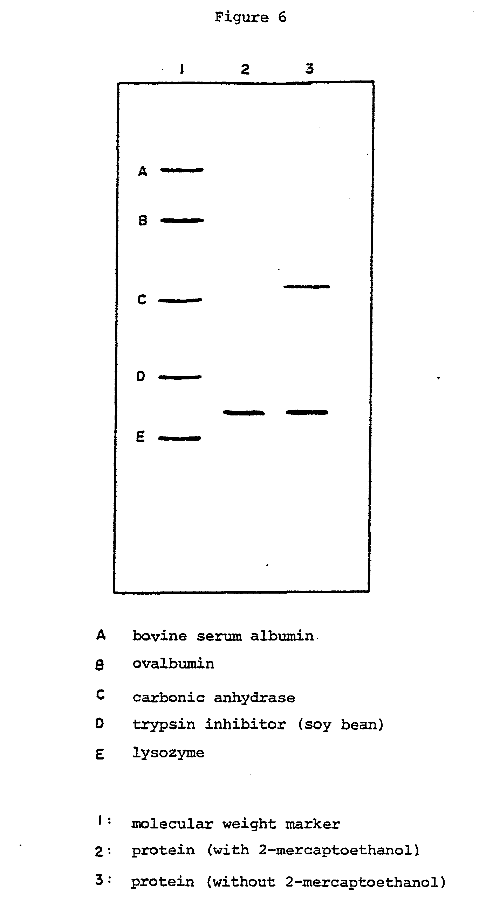

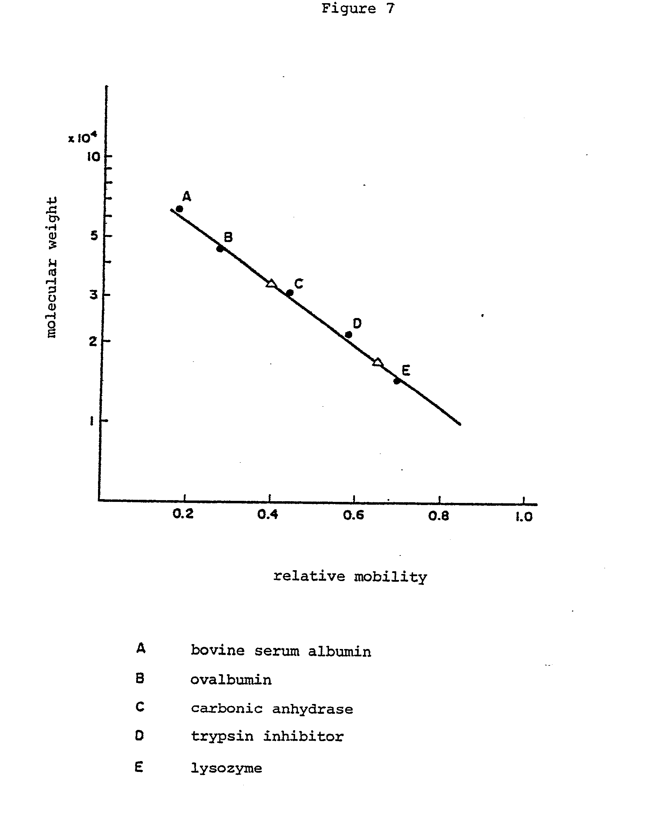

Fig. 1 is the restriction enzyme map for the plasmid pHIT3709 obtained in Example 1 (vii), in which the portion ▨▨▨▨ indicates the region coding for a peptide which is presumably the signal peptide and ◫◫◫ inidcates the region coding for the I-IF polypeptide. Fig. 2 illustrates the primary structure (base sequence) of the insert in the plasmid pHIT3709 obtained in Example 1 (vii), fig. 3 is the construction scheme for ptrp701 and ptrp771 described in Example 2 (i) and (ii), respectively, Fig. 4 is the construction scheme for pHITtrp1101 described in Example 2 (iii), and Fig. 5 is the construction scheme for pHITtrp2101 described in Example 2 (iv). Fig. 6 shows the results of the electrophoresis in Example 6 (i) and Fig. 7

0110044

the results of the molecular weight detemination in
Example 6 (i).

The present invention will be further explained by way
of the following working and reference examples, but these
examples should be understood not to limit thereby the
present invention.

Mouse B Hybridoma gamma 2-11.1 disclosed in the
following reference examples is deposited at C.N.C.M. of
Institut Pasteur, Paris, France, under the deposit number
I-242.

## Example 1

(i) Isolation of mRNA coding for human I-IF Lymphocytes prepared from the human peripheral blood were incubated at 37°C in the RPMI-1640 medium (containing 10% fetal calf serum) containing 15 ng/ml of 12-O-tetradecanoylphorbol-13-acetate (TPA) and 40 μg/ml of concanavalin A for I-IFN induction. After twenty-four (24) hours, the thus-induced human lymphocytes ($1 \times 10^{10}$ cells) were destructed in a thioguanidine solution (5M guanidine thiocyanate, 5% mercaptoethanol, 50 mM Tris HCl, pH 7.6, 10 mM EDTA) in a Teflon homogenizer. Then, sodium N-lauroyl sarcosinate was added in the concentration of 4% and the mixture after homogenization was layered over 6 ml of 5.7 M cesium chloride (5.7 M cesium chloride, 0.1 M ethylenediaminetetraacetate (EDTA)) and centrifuged at 15°C and 24000 rpm for 30 hours using a Beckman SW27 rotor to give a RNA precipitate. This RNA precipitate was dissolved in 0.25% sodium N-lauroyl sarcosinate and then precipitated with ethanol to give 8.3 mg of RNA. This RNA was allowed to be absorbed, in a high-concentration salt solution (0.5M-NaCl, 10 mM-Tris HCl; pH 7.6, 1 mM-EDTA, 0.3% SDS), on oligo (dT) cellulose column and mRNA containing poly(A) was eluted with a low-concentration salt solution (10 mM-Tris HCl, pH 7.6, 1 mM EDTA, 0.3% SDS) to given 700 μg of mRNA. This mRNA was further precipitated with ethanol, then dissolved in 0.2 ml of a solution (10 mM Tris HCl, pH 7.6, 2 mM EDTA, 0.3% SDS), treated at 65°C for 2 minutes, and fractionated by 10-35% sucrose density gradient centrifugation at 20°C and 25000 rpm for 21 hours using a Beckman SW27 rotor, to give 22 fractions. Aliquots of each fraction were injected

- 16 -

0110044

into *Xenopus laevis* oocytes and the proteins synthesized were assayed for interferon activity (antiviral activity as determined by the cytopathic effect inhibition assay using vesicular stomatitis virus on WISH cells derived from human amnion (Stewart, W.E. The interferon System, Springer New York, 1979, page 11)). In this manner, it was found that fraction 12 (the sedimentation constant being 12-14S) had an activity of 195 units per µg of RNA. The mRNA in the thus-obtained fraction 12 weighed about 20 µg.

(ii)  Synthesis of single-stranded RNA Using the above mRNA and a reverse transcriptase, 100 µl of a reaction mixture (5 µg of mRNA, 50 µg of oligo (dT), 100 units of reverse transcriptase, 1 mM each of dATP, dCTP, dGTP and dTTP, 8 mM $MgCl_2$, 50 mM KCl, 10 mM dithiothreitol and 50 mM Tris HCl; pH 8.3) was incubated at 42°C for 1 hour, then, deproteinized by adding phenol and treated with 0.1N NaOH at 70°C for 20 minutes for removal of RNA by decomposition.

(iii)  Synthesis of double-stranded DNA The thus-synthesized single stranded complementary DNA was subjected to reaction in 50 µl of a reaction mixture (the same mixture as above except that the mRNA and oligo dT were absent) at 42°C for 2 hours for synthesizing the double-stranded DNA.

(iv)  Addition of dC tails The double-stranded DNA was treated with nuclease S1  in 50 µl of a reaction mixture (double-stranded DNA, 0.1 M sodium acetate, pH 4.5, 0.25 M NaCl, 1.5 mM $ZnSO_4$, 60 units S1 nuclease) at room temperature for 30 minutes. The reaction mixture was deproteinized by adding phenol and DNA was precipitated with ethanol. The thus-obtained DNA was reacted with terminal transferase in 50 µl of a reaction mixture (double-stranded DNA, 0.14 M potassium cacodylate, 0.3 M Tris (base) (pH 7.6), 2 mM dithiothreitol, 1 mM

CoCl$_2$, 0.15 mM dCTP, 30 units terminal transferase) at 37°C for 3 minutes for elongation of the double-stranded DNA by about 20 deoxycytidine (about 20) units at each 3'-end of the DNA. This series of reactions gave about 300 ng of deoxycytidine-tailed double-stranded DNA.

(v) Cleavage of Escherichia coli plasmid and addition of dG tails Separately, 10 µg of Escherichia coli plasmid PBR 322 DNA was treated with restriction enzyme PstI in 50 µl of a reaction mixture (10 µg DNA, 50 mM NaCl, 6 mM Tris HCl (pH 7.4), 6 mM MgCl$_2$, 6 mM 2-mercaptoethanol, 100 µg/mg bovine serum albumin, 20 units PstI) at 37°C for 3 hours for cleavage at the one PstI recognition site present in the pBR322DNA, the reaction mixture was then deproteinized with phenol and the DNA was further subjected to terminal transferase treatment in 50 µl of a reaction mixture (10 µg DNA, 0.14 M potassium cacodylate, 0.3 M Tris (base) pH 7.6, 2 mM dithiothreitol, 1 mM CoCl$_2$, 0.15 mM dGTP, 30 units terminal transferase at 37°C for 3 minutes for elongation of the above pBR322 plasmid DNA by about 8 deoxyguanidines at each 3'-end thereof.

(vi) Annealing of cDNA and transformation of Escherichia coli The annealing was effected by heating 0.1 µg of the thus-obtained synthetic double-stranded DNA and 0.5 µg of the above pBR322 plasmid in a solution containing 0.1M NaCl, 50 mM Tris HCl pH 7.6, 1 mM EDTA at 65°C for 2 minutes and then at 45°C for 2 hours, followed by gradual cooling. The transformation of Escherichia coli X1776 was performed by the method of Enea et al. (J. Mol. Biol., 96, 495 (1975)).

(vii) Isolation of cDNA-containing plasmid About 8,500 tetracycline-resistant colinies were thus isolated and the DNA of each colony was fixed to a nitrocellulose filter (M. Grunstein and D.S. Hogness, Proc. Natl. Acad. Sci. USA, 72, 3961 (1975)).

Separately, based on the amino acid sequence of IF-γ as reported by D.V. Goeddel et al. (Nature, 295, 503 (1982)), two oligonuclotides of 5'TCCTGGCAGTAGC3' and 5'ACATTCATATCCTCCT3' presumably corresponding to amino acids Nos. 1-5 (Cys. Try. Cys. Gln. Asp) and amino acids Nos. 77-82 (Lys. Gln. Asp. Met. Asn. Val) of said I-IFN sequence, respectively, were chemically synthesized by the triester method (R. Crea et al., Proc. Natl. Acad. Sci. USA, 75, 5765 (1978)). These oligonucleotides were treated with T4 polynucleotide kinase in 50 μl of a reaction mixture (0.2 μg oligonucleotide, 50 mM Tris HCl pH 8.0, 10 mM MgCl$_2$ 10 mM mercaptoethanol, 50μCiγ-$^{32}$P ATP, 3 units T4 polynucleotide kinase) at 37°C for an hour. These oligopeptides thus labeled with $^{32}$P at the 5'-end were used as probes and annealed with the DNA on the above-mentioned nitrocellulose filter by the method of Lawn et al. (Nucleic Acids Res., 9, 6103 (1981)). Four strains were isolated by autoradiography which were reactive to the above two oligonucleotide probes.

Plasmid DNAs were isolated from the bacterial cells of each of these strains by the alkali method (H.C. Birnboim and J. Doly, Nucleic Acids Res., 7, 1513 (1979)). The inserts in the plasmid DNAs were excised with the PstI restriction enzyme. From among the isolated plasmids, the one containing the longest insert was chosen and named "pHIT3709".

The structure (base sequence) of the cDNA sequence inserted in the pHIT3709 plasmid was then determined by the dinucleotide synthetic chain termination method and by the Maxam-Gilbert method. Said primary structure was as shown in Fig. 2 (refer to Application No. 83 102 825.3).

The base sequence of the IF-γ coding region (codon No.31 to No.146) of pHIT3709 was identical with that shown in Fig.3 of Nucleic Acids Res.,10, 2487 et seq. (1982).

## Example 2

(1) Plasmid ptrp601 (vector being pBR322) containing the promoter portion for tryptophan synthesis in _Escherichia_ _coli_ (promotor- and operator-containing DNA fragment, 276 base pairs, G.N. bennett et al., J. Mol. Biol., _121_, 113 (1978)) was constructed as the expression plasmid.

Separately, plasmid pBR322 was cut with the restriction enzymes _EcoRI_ and _AvaI_. The thus obtained sticky ends of EcoRI-AvaI fragment which contained tetracycline-resistant gene were filled with DNA polymerase I large fragment. This fragment was allowed to be conjugated to the PvuII cleavage site of ptrp601 using T4DNA ligase. ptrp701 was thus constructed (Fig. 3).

(ii) For the purpose of excluding either of the two restriction enzyme _ClaI_ cleavage sites present in ptrp701, ptrp701 was subjected to partial decomposition treatment with _ClaI_ for giving ptrp701 in which either of the two _ClaI_ cleavage sites had been cut. After filling the sticky ends with DNA polymerase I large fragment, the thus-obtained ptrp701 was again conjugated using T4DNA ligase to give ptrp771 (Fig. 3).

(iii) pHIT3709 was cut with the restriction enzyme _PstI_ to give the _PstI_ fragment containing structural gene of IF-γ. This fragment was further subjected to the partial digetion with the restriction enzyme _BstNI_ to give the _BstNI_-_PstI_ fragment which was cut at the _BstNI_ site present in the IF-γ structural gene. The sticky ends of the _BstNI_ cleavage site were filled in with DNA polymerase I large fragment. The fragment thus obtained and the oligonucleotide adapter

CGATAATGTGTTACTGCC
TATTACACAATGACGG

, which was chemically synthesized by the above-mentioned triester method and contains the protein synthesis start codon ATG, were ligated using T4DNA ligase.

Separtely, the IF-γ gene ligated with the above-mentioned adapter was inserted into the PstI-ClaI site of Plasmid vector ptrp771 downstream from the tryptophan promoter using T4DNA ligase. An IF-γ expression plasmid pHITtrp1101 was thus constructed (Fig. 4).

(iv) pHITtrp2101 was constructed by further improvement of pHITtrp1101, as follows.

ptrp601 was first treated with the restriction enzymes ClaI and HpaII to give 0.33 Kb of the ClaI-HpaII fragment containing trp promoter. This fragment was ligated with pHITtrp1101, which was cut with ClaI and treated with alkaline phosphatase, using T4DNA ligase. pHITtrp2101 containing two successive trp promoters was thus obtained (Fig. 5).

A strain E. coli 294/pHIT·trp2101 was obtained by transforming Escherichia coli 294 with plasmid pHIT·trp2101 by the method of Cohen et al. (cit. supra).

### Example 3

E. Coli 294/pHITtrp2101 was incubated in 200 ml of M9 medium containing 8 μg/ml tetracycline, 0.4% casamino acid and 1% glucose in a 1-ℓ flask at 37°C. When the growth of the bacteria reached KU220, 3β-indolyl acrylic acid (IAA) was added at a concentration of 30 μg/ml and the mixture was further incubated for 4 hours.

### Example 4

After 1.2 ℓ of the culture solution as obtained in Example 3 was centrifuged, the cells were collected and suspended in 60 ml of 0.05 M Tris HCl (pH 7.6) containing 10% sucrose. To this cell suspension, 0.3 ml of 0.2 M phenylmethyl-sulfonyl fluoride (PMSF), 24 ml of 5M NaCl solution, 2.4 ml of 0.2 M ethylenediamine tetraacetate (EDTA), 2.4 ml of 1 M spermidine and 2.4 ml of lysozyme (5 mg/ml) were added. The mixture was allowed to stand at 0°C for 1 hour, incubated at 37°C for 5 minutes and, further, destructed at 0°C for 30 seconds using a ARTEK (USA) ultrasonic disintegrator.

This lysate solution was centrifuged at 105,000 x g for 1 hour for collecting 66 ml of the supernatant.

## Example 5

Sixty ml of the supernatant obtained in Example 4 was diluted with 20 mM Tris-HCl containing 1 mM EDTA and 0.15 M NaCl (pH 7.6) (TEN) to make 150 ml and the dilution was submitted to an anti-IF-γ antibody column (9 ml) prepared by the method of Ref. Example 10. The column was washed well with TEN and further with TEN containing 0.01% Nonidet P-40 (Shell) and 0.5 M NaCl. Then, IF-γ was eluted with 0.1 M acetic acid containing 0.25 M NaCl. The eluate was immediately neutralized with 1 M Tris-HCl (pH 7.6). The resulting solution was dialyzed against distilled water at 4°C for 16 hours and, after freezing with acetone-dry ice, lyophilized to give a powder. The results obtained are shown below.

| | Protein (mg) | Total activity (U) | Specific activity (U/mg) | Recovery (%) |
|---|---|---|---|---|
| Supernatant of lysate | 250.8 | $4 \times 10^8$ | $1.6 \times 10^6$ | - |
| After treatment on antibody column | 2.0 | $1.5 \times 10^8$ | $7.5 \times 10^7$ | 37.5 |

The specific activity of the final human immune interferon protein product thus obtained (based on the viral activity determination by the cytopathic effect inhibition assay using VSV and WISH cells (described preveiously herein)) was $7.5 \times 10^7$ U/mg. The characterization of this protein is mentioned below.

## Example 6

Characterization of human immune interferon protein
(i)   Molecular weight

The protein obtained in Example 5 was treated with 2-mercaptoethanol, subjected to SDS-polyacrylamide gel (17.5%) electrophoresis (15 mV, 6 hours) and stained with Coomassie blue. The protein could be identified as a single band (Fig. 6). From the relation between the migration distance for the molecular weight markers simultaneously subjected to electrophoresis and the migration distance for the protein, the molecular weight of the protein was estimated at 17,000 ± 1,000 (Fig. 7). For the protein not treated with 2-mercaptoethanol, an additional band was detected at a position corresponding to a molecular weight of 33,000 ± 2,000. This value is about two times the molecular weight of IF-γ namely 17,000 ± 1,000, suggesting that the band is due to dimerized IF-γ.

(ii) Amino acid analysis

An aliquot of the protein obtained in Example 5 was placed in a glass tube for hydrolysis and a 200 times (V/W) amount of constant boiling point hydrochloric acid containing 4% thioglycolic acid was added. The tube was sealed under reduced pressure and hydrolysis was conducted at 110°C for 24, 48 and 72 hours. After each hydrolysis, the tube was opened, the hydrochloric acid was removed under reduced pressure, and the residue was dissolved in 0.02 N hydrochloric acid and analyzed using a Hitachi Model 835 high-speed amino acid analyzer.

For the determination of cystine and cysteine, the above protein was oxidized with performic acid according to Hirs et al. (Methods in Enzymology, 11, 197, (1967)) and hydrolyzed in the same manner as above for 24 hours, and cysteinic acid was quantitatively determined by using the amino acid analyzer. The mean of three values obtained after 24, 48 and 72 hours of hydrolysis was employed as the found value for each amino acid except for the cases of serine, threonine, thyrosine and tryptophan, where the values were estimated by extrapolation of the hydrolysis period to 0 hour. The results are shown

in Table 2.

## Table 2

| Amino acid detected | Mole % | Amino acid detected | Mole % |
|---|---|---|---|
| Aspartic acid | 13.4 | Methionine | 2.9 |
| Threonine | 3.6 | Isoleucine | 4.8 |
| Serine | 7.4 | Leucine | 6.8 |
| Glutamic acid | 12.5 | Thyrosine | 3.4 |
| Proline | 1.4 | Phenylalanine | 6.7 |
| Glycine | 3.8 | Lysine | 13.5 |
| Alanine | 5.3 | Histidine | 1.5 |
| Cysteic acid | 1.3 | Arginine | 5.4 |
| Valine | 5.7 | Tryptophan | 0.9 |

(iii) Amino terminal amino acid analysis

The protein obtained in Example 13 was oxidized with performic acid according to Hirs et al. (Methods in Enzymology, 11, 197, (1967)) and then subjected to amino terminal amino acid analysis by the modified Edman degradation method of Iwanaga et al. (Eur. J. Biochem., 8, 189, (1969)). The resulting phenyl-thiohydantoin-amino acids (PTH-amino acids) were identified and quantitatively determined on a Varian (USA) Model 5040 high performance liquid chromatograph using an Ultra-sphere-ODS column (Altex, USA; 4.6 × 250 mm, particle size 5 μm) by the method of Archer et al. Altex Chromatogram, 3, 8, (1980)]. As a result, PTH-methionine sulfone and PTH-cysteic acid were detected.

As is evident from the above-mentioned examples, substantially pure human immune interferon proteins having a specific activity of not less than 7.5 x $10^7$ U/mg can be produced by the present invention.

## Example 7

Injectable preparation 5 mg of human I-IF protein of the present invention (specific activity of $2 \times 10^7$ U/mg) is dissolved in 100 ml of 5% HSA. The solution is filtered using membrane filter (pour size: 0.2 μm) and divided into 100 vials under aseptic conditions. The solution in vials are aseptically lyophirized to give injectable preparations for extemporaneous use.

The preparation is dissolved in 1 ml of distilled water before use.

In the following reference examples, thin layer chromatography was performed using Merck $60F_{254}$ silica gel plates or Funakoshi Yakuhin's AVICEL SF cellulose plates, with the developing solvents mentioned below:

$Rf^1$: Chloroform-methanol-acetic acid = 9:1:0.5

$Rf^2$: Ethyl acetate-pyridine-acetic acid-water = 30:10:3:5

$Rf^3$: Chloroform-methanol-water = 7:3:0.5

$Rf^4$: n-Butanol-pyridine-acetic acid-water = 30:20:6:24

$Rf^5$: Ethyl acetate-n-butanol-acetic acid-water = 1:1:1:1.

## Reference Example 1

(i)  Production of Z-Ser-Gln-OBu$^t$

Z-Gln-OBu$^t$ (10.1 g) was dissolved in 500 ml of methanol and catalytic reduction was carried out in a hydrogen gas stream using palladium black as the catalyst.  The catalyst was filtered off and the solvent was distilled off.  The residue was dissolved together with 7.5 g of Z-Ser-OH and 6.8 g of HONB in 250 ml of DMF and the solution was cooled with ice.  DCC (7.15 g) was added with ice-cooling and the mixture was stirred at 0°C for 4 hours and at room temperature for 12 hours. The formed DCU was filtered off and the solvent was distilled off.  The residue was extracted with 300 ml of AcOEt and the extract was washed with 4% aqueous NaHCO$_3$, 0.2 N hydrochloric acid and water in that order and dried over anhydrous sodium sulfate.  The solvent was then distilled off and the resulting crystalline precipitate was collected by filtration, dried and recrystallized from CH$_3$CN.  Yield 6.5 g (51.2%), mp 97-100°C, $(\alpha)_D^{25}$ -24.1° (c = 0.40, methanol)  Rf$^1$ 0.64.

Elemental analysis:
Calcd. for C$_{20}$H$_{29}$O$_7$N$_3$:
C, 56.72; H, 6.90; N, 9.92
Found:  C, 56.21; H, 6.72; N, 9.76

(ii)  Production of Z-Ala-Ser-Gln-OBu$^t$

Z-Ser-Gln-OBu$^t$ (4.23 g) was dissolved in 300 ml of methanol and catalytic reduction was carried out in a hydrogen gas stream using palladium black as the catalyst. The catalyst was filtered off and the solvent was distilled off.  The residue was dissolved together with 2.34 g of Z-Ala-OH and 2.27 g of HONB in a mixed solvent composed of 200 ml of AcOEt, 200 ml of dioxane and 100 ml of DMF and the solution was cooled with ice. DCC (2.38 g) was added with cooling and the mixture was stirred at 0°C for 4 hours and at room temperature for 12 hours.  The DCU was filtered off and the solvent was

distilled off. $CH_3CN$ and ether were added to the residue and the resulting crystalline precitate was collected by filtration, dried and recrystallized from $CH_3CN$. Yield 3.72 g (75.3%), mp 165-170°C, $[\alpha]_D^{25}$ -41.2° (c = 0.50, methanol) $Rf^1$ 0.60.

Elemental analysis:
Calcd. for $C_{23}H_{34}O_8N_4$:
C, 55.86; H, 6.93; N, 11.33
Found: C, 55.58; H, 6.74; N, 11.12

(iii) Production of Z-Arg(Pme)-Ala-Ser-Gln-OBu$^t$

Z-Ala-Ser-Gln-OBu$^t$ (3.46 g) was dissolved in 300 ml of methanol and catalytic reduction was carried out in a hydrogen gas stream using palladium black as the catalyst. The catalyst was filtered off and the solvent was distilled off. The residue was dissolved in 150 ml of DMF together with Z-Arg(Pme)-OH (prepared from 4.54 g of Z-Arg(Pme)-OH·CHA) and 1.9 g of HOBt, and the solution was cooled with ice. DCC (1.9 g) was added and the mixture was stirred at 0°C for 4 hours and at room temperature for 15 hours. The DCU was filtered off and the solvent was distilled off. AcOEt was added to the residue and the resulting precipitate was collected by filtration, dried and reprecipitated from methanol and AcOEt. Yield 4.55 g (75.5%), mp 130-134°C, $[\alpha]_D^{25}$ - 24.1° (c = 0.26, methanol) $Rf^1$ 0.57.

Elemental analysis:
Calcd. for $C_{40}H_{60}O_{11}N_8S \cdot \frac{1}{2}H_2O$:
C, 55.22; H, 7.07; N, 12.88; S, 3.69
Found: C, 55.23; H, 6.93; N, 12.54; S, 3.48

(iv) Production of Z-Arg(Pme)-Arg(Pme)-Ala-Ser-Gln-OBu$^t$

Z-Arg(Pme)-Ala-Ser-Gln-OBu$^t$ (4.3 g) was dissolved in 400 ml of methanol and catalytic reduction was carried out in a hydrogen gas stream using palladium black as the catalyst. The catalyst was filtered off and the solvent was distilled off. The residue was dissolved in 200 ml of DMF together with Z-Arg(Pme)-OH (prepared from 3.24 g of Z-Arg(Pme)-OH·CHA) and 1.42 g of HOBt and the solution was cooled with ice. DCC (1.41 g) was added and the mixture was stirred at 0°C for 4 hours and at room temperature for 20 hours. The DCU was filtered off and the solvent was distilled off. AcOEt was added to the residue and the resulting precipitate was collected by filtration, dried and reprecipitated from methanol and AcOEt. Yield 4.4 g (71.7%), mp 125-130°C, $(\alpha)_D^{25}$ - 18.0° (c = 0.40, methanol) Rf$^1$ 0.63.

Elemental analysis:

Calcd. for $C_{57}H_{86}O_{14}N_{12}S_2 \cdot H_2O$
C, 54.96; H, 7.12; N, 13.50; S, 5.15
Found: C, 54.66; H, 6.92; N, 13.32; S, 5.34

(v)  Production of Z-Arg(Pme)-Gly-OBu$^t$

Z-Gly-OBu$^t$ (13.0 g) was dissolved in 500 ml of MeOH and catalytic reduction was carried out in a hydrogen gas stream using palladium black as the catalyst. The catalyst was filtered off and the filtrate was concentrated under reduced pressure. The residue was dissolved in 200 ml of DMF, and Z-Arg(Pme)-OH (prepared from 20.0 g of Z-Arg(Pme)-OH·CHA) and 5.4 g of HOBt were added, followed by ice-cooling. DCC (8.2 g) was added and the mixture was stirred for 48 hours. The formed DCU was filtered off and the filtrate was concentrated. The residue was dissolved in 500 ml of AcOEt and the AcOEt solution was washed with 4% aqueous NaHCO$_3$ and 10% aqueous citric acid, dried over Na$_2$SO$_4$ and concentrated. Petroleum ether was added and the precipitate was collected by filtration. Yield 19.8 g (96.8%), mp 71-73°C, $(\alpha)_D^{23}$ + 0.2° (c = 0.9, DMF), Rf$^1$ 0.62.

Elemental analysis

Calcd. for $C_{31}H_{45}O_7N_5S$:
C, 58.93; H, 7.18; N, 11.09; S, 5.08
Found: C, 59.42; H, 7.58; N, 10.95; S, 4.84

(vi)  Production of Z-Phe-Arg(Pme)-Gly-OBu$^t$

Z-Arg(Pme)-Gly-OBu$^t$ (10.0 g) was dissolved in 500 ml of MeOH and catalytic reduction was carried out. The product was then dissolved in 300 ml of DMF. Z-Phe-OH (4.72 g) and 2.35 g of HOBt were added and the mixture was cooled with ice. DCC (3.59 g) was added and the whole mixture was stirred for 15 hours. The formed DCU was filtered off and the filtrate was concentrated. The residue was dissolved in 400 ml of AcOEt and the AcOEt solution was washed with 4% aqueous NaHCO$_3$ and 10% aqueous citric acid, dried over Na$_2$SO$_4$ and concentrated. Ether was added and the resulting crystalline precipitate was collected by filtration and recrystallized from MeOH-ether. Yield 10.5 g (85.3%), mp 101-103°C, $(\alpha)_D^{26}$ - 8.3° (c = 0.9, DMF), Rf$^1$ 0.64.

Elemental analysis

Calcd. for C$_{40}$H$_{54}$O$_8$N$_6$S:

C, 61.67; H, 6.99; N, 10.79; S, 4.12 Found: C, 61.66; H, 6.56; N, 10.93; S, 4.14

(vii) Production of Z-Leu-Phe-Arg(Pme)-Gly-OBu$^t$

Z-Phe-Arg(Pme)-Gly-OBu$^t$ (5.5 g) was catalytically reduced in 300 ml of MeOH and then dissolved in 300 ml of DMF. Z-Leu-OH (prepared from 3.31 g of Z-Leu-OH·DCHA) and 1.47 g of HONB were added and the mixture was cooled with ice. DCC (1.68 g) was added and the whole mixture was stirred for 48 hours. The formed DCU was filtered off and the filtrate was concentrated. The residue was dissolved in 300 ml of AcOEt and the AcOEt solution was washed with 4% aqueous NaHCO$_3$ and 10% aqueous citric acid, dried over Na$_2$SO$_4$ and concentrated. Ether was added and the precipitate was collected by filtration. Yield 6.2 g (98.3%), mp 171-173°C, $(\alpha)_D^{26}$ - 15.5° (c = 0.9, DMF), Rf$^1$ 0.64.

Elemental analysis

Calcd. for C$_{46}$H$_{65}$O$_9$N$_7$S:

C, 61.93; H, 7.34; N, 10.99; S, 3.59 Found: C, 62.02; H, 7.37; N, 11.08; S, 3.59

(viii) Production of Boc-Met-Leu-Phe-Arg(Pme)-Gly-OBu$^t$

Z-Leu-Phe-Arg(Pme)-Gly-OBu$^t$ (6.0 g) was catalytically reduced in 200 ml of MeOH and then dissolved in 150 ml of DMF. Boc-Met-OH (prepared from 3.0 g of Boc-Met-OH·DCHA) and 1.39 g of HONB were added and the mixture was cooled with ice. DCC (1.59 g) was added and the whole mixture was stirred for 15 hours. The formed DCU was filtered off and the filtrate was concentrated. The residue was dissolved in n-BuOH-AcOEt and the solution was washed with 10% aqueous citric acid, dried over Na$_2$SO$_4$ and concentrated. Ether was added and the crystals was collected by filtration. Yield 6.2 g (93.1%), mp 192-195°C, $(\alpha)_D^{26}$ - 19.7° (c = 1.0, DMF), Rf$^1$ 0.64.

Elemental analysis

Calcd. for C$_{48}$H$_{76}$O$_{10}$N$_8$S$_2$:

C, 58.27; H, 7.74; N, 11.33; S, 6.48 Found: C, 58.48; H, 7.79; N, 11.34; S, 5.98

(ix) Production of Boc-Gln-Met-Leu-Phe-Arg(Pme)-Gly-OH

To 5.5 g of Boc-Met-Leu-Phe-Arg(Pme)-Gly-OBu$^t$ was added 50 ml of TFA and the mixture was shaken at room temperature for 10 minutes and then concentrated. Ether was added and the precipitate was collected by filtration and dried. It was dissolved in 50 ml of DMF and the solution was cooled with ice, followed by addition of 1.8 ml of TEA. Boc-Gln-ONB (prepared from 1.85 g of Boc-Gln-OH, 1.49 g of HONB and 1.90 of DCC) was added and the mixture was stirred for 15 hours and concentrated. AcOH and then AcOEt were added and the resulting precipitate was collected by filtration. Yield 5.3 g (89.8%), mp 181-183°C (decomp.), $(\alpha)_D^{26}$ - 19.6° (c = 1.0, DMF), Rf$^1$ 0.30.

Elemental analysis

Calcd. for C$_{49}$H$_{76}$O$_{12}$N$_{10}$S$_2$:

C, 55.45; H, 7.22; N, 13.20; S, 6.04 Found: C, 55.39; H, 7.17; N, 13.34; S, 6.20

(x) Production of Z-Ser-NHNH-Boc

Z-Ser-OH (10.0 g) and 6.2 g of t-butyl carbazate were dissolved in 150 ml of DMF and the solution was ice-cooled. HONB (8.0 g) and 9.3 g of DCC were added and the mixture was stirred for 15 hours. The formed DCU was filtered off and the filtrate was concentrated. The residue was extracted into AcOEt and the AcOEt solution was washed with 4% aqueous $NaHCO_3$ and 10% aqueous citric acid, dried over $Na_2SO_4$ and concentrated. Ether was added and the crystals was collected by filtration. Yield 7.3 g (50.4%), mp 95-98°C, $(\alpha)_D^{26}$ - 6.2° (c = 0.8, DMF), $Rf^1$ 0.62.

Elemental analysis

Calcd. for $C_{16}H_{23}O_6N_3$:
C, 54.38; H, 6.56; N, 11.89
Found: C, 54.77; H, 6.88; N, 12.29

(xi) Production of Z-Arg(Pme)-Ser-NHNH-Boc

Z-Ser-NHNH-Boc (3.9 g) was catalytically reduced in 300 ml of MeOH and then dissolved in 50 ml of DMF. Z-Arg(Pme)-OH (prepared from 6.2 g of Z-Arg(Pme)-OH·CHA) and 1.5 g of HOBt were added and the mixture was ice-cooled. DCC (2.3 g) was added and the whole mixture was stirred for 15 hours. The formed DCU was filtered off and the filtrate was concentrated. The residue was dissolved in AcOEt and the AcOEt solution was washed with 4% aqueous $NaHCO_3$ and 10% aqueous citric acid, dried over $Na_2SO_4$ and concentrated. Ether was added and the resulting precipitate was collected by filtration. Yield 7.45 g (93.7%), mp 100-101°C, $(\alpha)_D^{26}$- 0.9) (c = 1.2, DMF), $Rf^1$ 0.51.

Elemental analysis

Calcd. for $C_{33}H_{49}O_9N_7S$:
C, 55.06; H, 6.86; N, 13.62; S, 4.46
Found: C, 55.49; H, 6.94; N, 13.14; S, 3.86

(xii) Production of Z-Lys(Mtr)-Arg(Pme)-Ser-NHNH-Boc

Z-Arg(Pme)-Ser-NHNH-Boc (3.9 g) was dissolved in 300 ml of MeOH and catalytic reduction was carried out.

The product was dissolved in 50 ml of DMF, and Z-Lys(Mtr)-OH (prepared from 3.4 g of Z-Lys(Mtr)-OH·DCHA) and 0.88 g of HOBt were added. The mixture was ice-cooled and 1.34 g of DCC was added. The whole mixture was stirred for 20 hours. The formed DCU was filtered off and the filtrate was concentrated. AcOEt was added and the powdery precipitate was collected by filtration and recrystallized from MeOH-AcOEt. Yield 5.1 g (93.4%), mp 103-105°C, $(\alpha)_D^{26} - 7.2°$ (c = 0.8, DMF), $Rf^1$ 0.57.

Elemental analysis

Calcd. for $C_{49}H_{73}O_{13}N_9S_2$:

C, 55.50; H, 6.94; N, 11.89; S, 6.05 Found: C, 55.70; H, 7.15; N, 11.60; S, 5.63

(xiii) Production of Z-Arg(Pme)-Lys(Mtr)-Arg(Pme)-Ser-NHNH-Boc

Z-Lys(Mtr)-Arg(Pme)-Ser-NHNH-Boc (4.8 g) was dissolved in 300 ml of MeOH and catalytic reduction was carried out. The product was dissolved in 70 ml of DMF, and Z-Arg(Pme)-OH (prepared from 2.8 g of Z-Arg(Pme)-OH·CHA) and 0.74 g of HOBt were added. The mixture was ice-cooled and 1.12 g of DCC was added. The whole mixture was stirred for 15 hours. The formed DCU was filtered off and the filtrate was concentrated. The residue was dissolved in AcOEt and the AcOEt solution was washed with 4% aqueous $NaHCO_3$ and 10% aqueous citric acid, dried over $Na_2SO_4$ and concentrated. Ether was added and the resulting precipitate was collected by filtration and reprecipitated from MeOH-ether. Yield 5.8 g (89.8%), mp 136-137°C, $(\alpha)_D^{26} - 6.6°$ (c = 1.0, DMF), $Rf^1$ 0.58.

Elemental analysis

Calcd. for $C_{66}H_{99}O_{16}N_{13}S_3$:

C, 55.56; H, 6.99; N, 12.76; S, 6.74 Found: C, 55.34; H, 7.07; N, 12.50; S, 6.59

(xiv) Production of Z-Lys(Mtr)-Arg(Pme)-Lys(Mtr)-Arg(Pme)-Ser-NHNH-Boc

Z-Arg(Pme)-Lys(Mtr)-Arg(Pme)-Ser-NHNH-Boc (3.0 g) was dissolved in 150 ml of MeOH and catalytic reduction was carried out. The product was dissolved in 60 ml of DMF, and Z-Lys(Mtr)-OH (prepared from 1.54 g of Z-Lys(Mtr)-OH·DCHA) and 0.31 g of HOBt were added. The mixture was ice-cooled and 0.57 g of DCC was added. The whole mixture was stirred for 15 hours. The formed DCU was filtered off and the filtrate was concentrated. The residue was dissolved in AcOEt and the AcOEt solution was washed with 4% aqueous NaHCO$_3$ and 10% aqueous citric acid, dried over Na$_2$SO$_4$ and concentrated. Ether was added and the crystalline precipitate was collected by filtration and recrystallized from AcOEt. Yield 2.70 g (72.8%), mp 123-125°C, $(\alpha)_D^{26}$ - 5.9° (c = 1.1, DMF), Rf$^1$ 0.57.

Elemental analysis

Calcd. for C$_{82}$H$_{123}$O$_{20}$N$_{15}$S$_4$:

C, 55.73; H, 7.02; N, 11.89; S, 7.26 Found: C, 55.89; H, 7.30; N, 11.72; S, 7.08

(xv) Production of Boc-Gln-Met-Leu-Phe-Arg(Pme)-Gly-Arg(Pme)-Arg(Pme)-Ala-Ser-Gln-OBu$^t$

Z-Arg(Pme)-Arg(Pme)-Ala-Ser-Gln-OBu$^t$ (1.0 g) was dissolved in 100 ml of MeOH and catalytic reduction was carried out. The product was then dissolved in 20 ml of DMF, and 0.85 g of Boc-Gln-Met-Leu-Phe-Arg(Pme)-Gly-OH and 135 mg of HOBt were added. The mixture was ice-cooled and 210 mg of DCC was added. The whole mixture was stirred for 20 hours. The formed DCU was filtered off and the filtrate was concentrated. MeOH was added and the resulting precipitate was collected by filtration. Yield 1.50 g (87.4%), mp 216-217°C (decomp.), $(\alpha)_D^{26}$ - 11.8° (c = 1.0, DMF), Rf$^1$ 0.43.

Elemental analysis

Calcd. for C$_{98}$H$_{154}$O$_{23}$N$_{22}$S$_4$:

C, 55.09; H, 7.27; N, 14.42; S, 6.00 Found: C, 54.81; H, 7.33; N, 14.23; S, 5.79

(xvi)  Production of H-Lys-Arg-Lys-Arg-Ser-Gln-Met-Leu-
Phe-Arg-Gly-Arg-Arg-Ala-Ser-Gln-OH

To 1.0 g of Boc-Gln-Met-Leu-Phe-Arg(Pme)-Gly-
Arg(Pme)-Arg(Pme)-Ala-Ser-Gln-OBu$^t$ was added 10 ml of
TFA and the mixture was shaken at room temperature for
50 minutes and then concentrated. Ether was added and
the precipitate was collected by filtration and dried.

Separately, 10 ml of TFA was added to 0.83 g
of Z-Lys(Mtr)-Arg(Pme)-Lys(Mtr)-Arg(Pme)-Ser-NHNH-Boc
and the mixture was shaken at room temperature for 10
minutes and then concentrated. Ether was added and the
precipitate was collected by filtration and dried.
It was dissolved in 10 ml of DMF and the solution was
cooled with dry ice-acetone, followed by addition of
0.23 ml of 6.2 N HCl/AcOEt and isoamyl nitrite (0.075 ml).
The temperature was maintained at -25° to -20°C for
20 minutes (negative to the hydrazine test) and the
reaction mixture was cooled again with dry ice-acetone and
then neutralized with 0.25 ml of TEA. The amine component
was dissolved in 40 ml of DMF and the solution was
ice-cooled. TEA (0.16 ml) was added and the above
azide solution was added. The whole mixture was stirred
at 4°C for 72 hours and poured into diluted acetic acid.
The formed precipitate was collected by filtration and
washed with aqueous CH$_3$CN. Yield 1.40 g (81.5%),
Rf$^1$ 0.09. A part (400 mg) of this product was dissolved
in 60 ml of TFA-thioanisole-methyl sulfide (8:1:1)
containing 0.15 M MSA and the solution was shaken at
room temperature for 2 hours. AcONH$_4$ (400 mg) was added
and the mixture was concentrated. Ether was added and the
precipitate was collected by filtration and dried.
It was dissolved in a small amount of 1 N AcOH and the
solution was passed through a Sephadex G-25 column
(2.2 × 120 cm) using 1 N AcOH as the eluent. Fractions from
160 ml to 260 ml were combined and lyophilized. The
lyophilizate was then passed through a column of Amberlite IRA-410 (acetate form) and lyophilized.
This lyophilizate was purified by HPLC using a TSK-LS-
410 column (2.14 × 7.5 cm + 2.14 × 30 cm) to give the
above-identified compound. Yield 45 mg, $(\alpha)_D^{24}$ - 45.9°
(c = 0.7, 0.1 N AcOH), $Rf^4$ (cellulose) 0.20.

Amino acid analysis: Lys 1.71, Arg 4.71, Ser 1.69,
Glu 2.12, Gly 1.00, Ala 1.03, Met 0.32, Leu 1.30, Phe
1.08 (average recovery rate 77%).

Reference Example 2

Synthesis of carrier protein-polypeptide complex

The polypeptide obtained in Ref. Example 1 (xvi) was
coupled with thyroglobulin (hereinafter, TG) according
to Goodfriend et al. [Sceince, 144, 1334
(1964)]. Thus, 2.5 mg of said polypeptide was mixed with 3.75
mg of TG and, following addition of 2 ml of 50 mM
phosphate buffer, the mixture was stirred well in ice water.
Thereto was gradually added drop by drop a solution of
30.4 mg of carbodiimide hydrochloride in 200 ml of distilled
water. Thereafter, the mixture was stirred in ice-water
for 3 hours. After the reaction, dialysis was performed
against distilled water to a sufficient extent, followed
by lyophilization to give 4.7 mg of a protein complex.

Reference Example 3

Preparation of enzyme-linked antigen for antibody
detection by EIA

The enzyme-linked antigen for EIA was prepared
according to Kitagawa et al. (Journal of Biochemistry,
79, 233, (1976)].

(i)  Introduction of a maleimido group into the
     polypeptide

The polypeptide (350 nmoles) as obtained in Ref. Example
1 (xvi) was dissolved in 1 ml of 100 mM phosphate buffer
(pH 6.8, and the solution was added to a solution of 585
μg (1.75 μmoles) of N-(4-carboxycyclohexylmethyl)maleimide N-hydroxysuccinimide ester in 70 μl of N,N-dimethylformamide. The mixture was stirred at 30°C for 30 minutes. After the reaction, fractionation was performed using a Sephadex G-25 column to give 185 nmoles of a polypeptide fraction with the maleimido group introduced therein.

(ii) Coupling of the maleimido-group-containing polypeptide with β-D-galactosidase

The maleimido-containing polypeptide (16.5 nmoles) as obtained in Ref. Example 3 (i) was mixed with 3.3 nmoles of β-D-galactosidase. After 18 hours of reaction at 4°C, 412.5 nmoles of β-mercaptoethanol was added for teminating the reaction. The β-D-galactosidase-coupled polypeptide was fractionated on a Sepharose 6B column and used for the subsequent experiments.


Reference Example 4

EIA method

The detection of antibody activity in the serum of mice immunized with the protein complex obtained in Ref. Example 2 or in the hybridoma supernatant was conducted by the EIA method (Immunology, 1, 3, (1978)). Thus, the serum or hybridoma supernatant was diluted with buffer A (20 mM $Na_2HPO_4$, 100 mM NaCl, 0.1% $NaN_3$, 1 mM $MgCl_2$, pH 7.0), a 100-μl portion of the dilution was mixed with 100 μl of the polypeptide derivative as obtained in Example 3, and the reaction was allowed to proceed at 24°C for 24 hours. Thereafter, 100 μl of 3% cellulose coupled with rabbit anti-mouse IgG was added, and the reaction was allowed to proceed at 24°C for 4 hours. After the reaction, the cellulose was washed well with buffer A containing 0.5% of Tween 20, then 500 μl of 20 μg/ml 4-methylumbelliferyl-β-D-galactoside was added and, after 2 hours of reaction at 37°C, 3 ml of 100 mM carbonate buffer (pH 10.5) was added for terminating the reaction. The fluorescence intensity was measured with a fluorometer (excitation:

365 nm; emission: 450 nm).


## Reference Example 5

Immunization

Each of 6 female BALB/C mice aged 7 to 8 weeks was subcutaneously inoculated with 40 µg (on the protein basis) of the protein complex obtained in Ref. Example 2 (as the antigen in intimate admixture with Freund's complete adjuvant (primary immunization). Two weeks after the primary immunization, the mice were subcutaneously inoculated with the antigen at the same dose as above in intimate admixture with Freund's incomplete adjuvant (secondary immunization). Further two weeks later, a third immunization was made in the same manner as in the secondary immunization. Six days after the third immunization, partial blood sampling was made from the mice and the serum antibody titers were determined by the EIA method described in Ref. Example 4. The mouse numbered γ-2 gave the highest antibody titer and subjected to the final immunization by intravenous inoculation with 120 µg of the antigen dissolved in 0.5 ml of aqueous sodium chloride. The antibody titer data for each mouse are shown in Table 3.

Table 3  Antipeptide antibody titers
in immunized mice

| Mouse No. | B/T (%) | | |
| | Primary immunization 1) | Secondary immunization 2) | Third immunization 3) |
|---|---|---|---|
| γ-1 | − 4) | N.D | 24.5 |
| 2 | N.D 5) | 19.3 | 35.3 |
| 3 | − | N.D | 24.7 |
| 4 | N.D | 1.3 | 1.7 |
| 5 | N.D | 1.8 | 5.0 |
| 6 | − | N.D | 0.8 |
| Normal mouse | 0.6 | 0.1 | N.D |

1) Serum dilution ratio: 1/1000
2) Serum dilution ratio: 1/6300
3) Serum dilution ratio: 1/7800
4) -: Not detectable
5) ND: Not determined

B/T: (Bound enzyme activity/total added enzyme
activity) × 100


Reference Example 6

Cell fusion

Immunization was performed by the method described in Ref. Example 5. Three days after the final immunization, the spleen was excised from the γ-2 mouse, filtered under pressure through a stainless mesh, and suspended in Eagle's minimum essential medium (MEM) to give a spleen cell suspension. For cell fusion, BALB/C mouse-derived P3-x63.Ag8.U1 (P3U1) myeloma cells were used (Current Topics in Microbiology and Immunology, 81, 1, (1978)). Cell fusion was performed by the original method (Nature, 256, 495, (1975)). Thus, spleen cells and P3U1 cells were separately washed three times with serum-free MEM and mixed at a ratio of 5:1 (in number of cells). The mixture was centrifuged at 800 rpm for 15 minutes, whereby the cells were settled. After thorough removal of the supernatant, the sediment was lightly loosened, 0.3 ml of 45% polyethylene glycol (PEG) 6000 (Koch-Light) was added, and the mixture was allowed to stand in a warm water tank maintained at 37°C for 7 minutes so as to effect cell fusion. Thereafter, MEM was added thereto at a rate of 2 ml per minute. After addition of 12 ml in total of MEM, the resulting mixture was centrifuged at 600 rpm for 15 minutes, followed by removal of the supernatant. The cell sediment was suspended in RPMI-1640 medium supplemented with 10% fetal calf serum (RPMI1640-10FCS) in a concentration of $2 \times 10^5$ P3U1 cells/ml and each of 144 wells on 24-well multi-dishes (Linbro) was seeded with 1 ml of the suspension.

After seeding, the cells were incubated at 37°C in a 5% carbon dioxide gas incubator. After 24 hours, HAT-selective culture was started by adding PRMI1640-10FCS medium supplemented with HAT ($1 \times 10^{-4}$ M hypoxanthine, $4 \times 10^{-7}$ M aminopterin, $1.6 \times 10^{-5}$ M thymidine) (HAT medium) in an amount of 1 ml per well. The HAT-selective culture was continued while 1 ml of the old medium was replaced by 1 ml of fresh HAT medium 3, 5 and 7 days after start of the culture. The growth of hybridomas was noted 10 to 14 days after cell fusion. When the culture broth turned yellow (about $1 \times 10^{6}$ cells/ml), the supernatant was collected and examined for the presence of antibody by the EIA method. In this manner, supernatants from 141 wells in which hybridoma growth had been noted were examined. Two wells ($\gamma2$-11 and $\gamma2$-100) afforded intense antibody activity and other two wells ($\gamma2$-62 and $\gamma2$-70) presented weak antibody activity.

## Reference Example 7

Cloning

Hybridomas from 3 wells ($\gamma2$-11, 62 and 100) which were positive in antibody activity were cloned by the limiting dilution method. Thus, hybridomas were suspended in RPMI1640-20FCS in a concentration of 2 hybridomas/ml and the suspension was distributed in 0.1-ml portions into the wells on a 96-well microplate (Nunc). In said distribution, $5 \times 10^{5}$ per well of BALB/c mouse thymocytes were added as feeder cells. As a result, cell proliferation was observed in about 2 weeks. The supernatant was then collected and examined for the presence of antibodies by the EIA method as described in Example 4. Antibody activity was noted in 8 out of 19 clones from $\gamma2$-11 in 3 out of 54 clones from $\gamma2$-62 and in 5 out of 47 clones from $\gamma2$-100.

Reference Example 8

Ascites formation by monoclonal antibody-producing
hybridomas

Ascites formation was caused by intraperitoneal
inoculation of BALB/c mice intraperitoneally pretreated
with 0.5 ml of mineral oil with $1 \times 10^6$ γ2-11.1 clone
cells capable of producing antibodies having IFN-γ-
binding activity. Ten days after intraperitoneal
administration of hybridomas, the ascitic fluid was
taken and examined for antibody activity up to $10^7$-fold
dilution. While the antibody activity of the corresponding
clone cell culture supernatant was detected up to
$10^4$-fold dilution, the formation of ascites (ascitization)
led to an about 1000 times increase in antibody activity.

Reference Example 9

Monoclonal antibody purification

Using 4 ml of the ascitic fluid obtained in Ref. Example
8 as the starting material, monoclonal antibody purification
was performed by the method of Staehelin et al.
[Journal of Biological Chemistry, 256, 9750,
(1981)]. Thus, the ascitic fluid was first centrifuged at
10,000 rpm for 15 minutes to remove fibrin-like substances
therefrom and then diluted with phosphate buffer-saline
(PBS: 8.1 mM $NaH_2PO_4$, 1.5 mM $KH_2PO_4$, 2.7 mM KCl, 137 mM
NaCl; pH 7.2) to a concentration at which the ultraviolet
absorption at 280 nm ($A_{280}$) for said dilution would range
from 12 to 14. Thereafter, saturated aqueous ammonium
sulfate was added to the diluted sample to a concentration
of 47% for the sulfate. The mixture was stirred at 4°C
for 60 minutes to effect salting out and then centrifuged
(10,000 rpm, 15 minutes) to give a precipitate.
The precipitate was dissolved in 20 mM Tris buffer (pH
7.9) containing 50 mM NaCl and dialyzed against 2 liters
of the same buffer. Tow hours later, the dialyzing
solution was replaced by a fresh 2-liter portion of the same solution and the dialysis was continued for further 15 hours. Thereafter, the precipitate was removed by centrifugation at 10,000 rpm for 15 minutes, and the supernatant was adjusted to a concentration such that the $A_{280}$ value became 20-30. This sample was subjected to fractionation on a DEAE-cellulose column (8 ml, Whatman $DE_{52}$) equilibrated with a sufficient amount of Tris buffer containing 50 mM NaCl. After extensive washing with the same buffer at a flow rate of 1.5 ml/min, NaCl consentration was linearly increased from 50 mM to 500 mM. Under these conditions, the antibody activity was detected mainly in effluent fractions.

## Reference Example 10

Twenty-five ml (65.3 mg) of the monoclonal antibody from the effluent fractions as purified by the procedure of Ref. Example 9 was dialyzed overnight against 0.1 M $NaHCO_3$ (pH 8.3). Separately, 25 ml of AFFI-GEL 10 (Bio-Rad) was thoroughly washed with water using a glass filter, suspended in 0.1 M $NaHCO_3$ (pH 8.3) and mixed with the above antibody. The mixture was stirred gently at 4°C for 4 hours to effect the reaction, and then allowed to stand at 4°C overnight. The resulting AFFI-GEL 10 was washed well with 0.1 M $NaHCO_3$ (pH 8.3) using a glass filter. To the gel was added 25 ml of a solution (pH 8.0) containing 0.1 M ethanolamine and 0.15 M NaCl. The mixture was shaken at 4°C for an hour so as to block possibly remaining unreacted active groups. Then, the gel was washed well with PBS, and suspended in 25 ml of 0.1% $NaN_3$-containing PBS. The suspension was stored at 4°C. Based on the amount of the added antibody and the amount of the antibody in the recovered filtrate, it was found that the antibody was conjugated to the gel in a proportion of 2.35 mg/ml of gel. A column was packed with the reaction product obtained in this manner and used as an antibody column.

What is claimed is:

1.    A substantially pure reconbinant human immune interferon protein.

2.    The protein according to claim 1, which has a specific activity of not less than $10^7$ U/mg.

3.    The protein according to claim 1 or 2, which is in a lyophilized form.

4.    A method for producing a substantially pure recombinant human immune interferon protein, which comprises growing a transformant having DNA containing a base sequence encoding human immune interferon to produce and accumulate human immune interferon in the transformant cells, lysing the cells and purifing the resulting human immune interferon-containing solution with use of monoclonal antibody capable of binding to human immune interferon.

5.    The method according to claim 4, wherein the recombinant human immune interferon protein has a specific activity of not less than $10^7$ U/mg.

6.    The method according to claim 4, wherein the base sequence encoding human immune interferon is of the formula:
(5') TGT TAC TGC CAG GAC CCA TAT GTA AAA GAA
GCA GAA AAC CTT AAG AAA TAT TTT AAT GCA GGT
CAT TCA GAT GTA GCG GAT AAT GGA ACT CTT TTC
TTA GGC ATT TTG AAG AAT TGG AAA GAG GAG AGT
GAC AGA AAA ATA ATG CAG AGC CAA ATT GTC TCC
TTT TAC TTC AAA CTT TTT AAA AAC TTT AAA GAT
GAC CAG AGC ATC CAA AAG AGT GTG GAG ACC ATC
AAG GAA GAC ATG AAT GTC AAG TTT TTC AAT AGC

```
AAC AAA AAG AAA CGA GAT GAC TTC GAA AAG CTG
ACT AAT TAT TCG GTA ACT GAC TTG AAT GTC CAA
CGC AAA GCA ATA CAT GAA CTC ATC CAA GTG ATG
GCT GAA CTG TCG CCA GCA GCT AAA ACA GGG AAG
CGA AAA AGG AGT CAG ATG CTG TTT CGA GGT CGA
AGA GCA TCC CAG-X (3')
```

wherein X is TAA, TGA or TAG.

7.    The method according to claim 4, wherein the monoclonal antibody is one against the polypeptide of the formula:

(N)                                          (C)
H-X-Lys Arg Ser Gln Met Leu Phe Arg Gly-Y-OH wherein X is a bond, or a peptide or amino acid residue having 1 to 16 amino acids counting from the C terminus of the peptide chain of

(N)
Ile Gln Val Met Ala Glu Leu Ser Pro Ala
                           (C)
Ala Lys Thr Gly Lys Arg and Y is a peptide or amino acid residue having 1 to 5 amino acids counting from the N terminus of the peptide chain of

(N)              (C)
Arg Arg Ala Ser Gln.

8.    The method according to claim 7, wherein Y is Arg Arg Ala Ser Gln.

9.    The method according to claim 7 or 8, wherein X is Lys Arg.

10.    A pharmaceutical composition which contains an effective amount of a substantially pure recombinant human immune interferon protein and a pharmaceutically acceptable carrier, vehicle or diluent therefor.

11.    The composition according to claim 10, wherein the recombinant human immune interferon protein has a specific activity of not less than $10^7$ U/mg.

12.    The composition according to claim 10, which is an injectable preparation.

13.    A method for producing a pharmaceutical composition containing an effective amount of a substantially pure recombinant human immune interferon protein which comprises admixing said human immune interferon protein with a pharmaceutically acceptable carrier, vehicle or diluent therefor.

14.    The method according to claim 13, wherein the recombinant human immune interferon protein has a specific activity of not less than $10^7$ U/mg.

15.    The method according to claim 13, wherein the composition is an injectable preparation.

Figure 1

S1
5' ACTTCTTTGGCTTAATTCTCTCGGAAACG ATG AAA TAT ACA AGT TAT ATC TTG

S20  1
GCT TTT CAG CTC TGC ATC GTT TTG GGT TCT CTT GGC TGT TAC TGC CAG

20
GAC CCA TAT GTA AAA GAA GCA GAA AAC CTT AAG AAA TAT TTT AAT GCA

GGT CAT TCA GAT GTA GCG GAT AAT GGA ACT CTT TTC TTA GGC ATT TTG

40
AAG AAT TGG AAA GAG GAG AGT GAC AGA AAA ATA ATG CAG AGC CAA ATT

60
GTC TCC TTT TAC TTC AAA CTT TTT AAA AAC TTT AAA GAT GAC CAG AGC

80
ATC CAA AAG AGT GTG GAG ACC ATC AAG GAA GAC ATG AAT GTC AAG TTT

100
TTC AAT AGC AAC AAA AAG AAA CGA GAT GAC TTC GAA AAG CTG ACT AAT

TAT TCG GTA ACT GAC TTG AAT GTC CAA CGC AAA GCA ATA CAT GAA CTC

120
ATC CAA GTG ATG GCT GAA CTG TCG CCA GCA GCT AAA ACA GGG AAG CGA

140            146
AAA AGG AGT CAG ATG CTG TTT CGA GGT CGA AGA GCA TCC CAG TAA TGG

TTGTCCTGCCTGCAATATTTGAATTTTAAATCTAAATCTATTTATTAATATTTAACATTATTT

ATATGGGGAATATATTTTTAGACTCATCAATCAAATAAGTATTTATAATAGCAACTTTTGTGT

AATGAAAATGAATATCTATTAATATATGTATTATTTATAATTCCTATATCCTGTGACTGTCTC

ACTTAATCCTTTGTTTTCTGACTAATTAGGCAAGGCTATGTGATTACAAGGCTTTATCTCAGG

GGCCAACTAGGCAGCCAACCTAAGCAAGATCCCATGGGTTGTGTGTTTATTTCACTTGATGAT

ACAATGAACACTTATAAGTGAAGTGATACTATCCAGTTACTGCCGGTTTGAAAATATGCCTGC

AATCTGAGCCAGTGCTTTAATGGCATGTCAGACAGAACTTGAATGTGTCAGGTGACCCTGATG

AAAACATAGCATCTCAGGAGATTTCATGCCTGGTGCTTCCAAATATTGTTGACAACTGTGACT

GTACCCAAATGGAAAGTAACTCATTTGTTAAAATTATCAATATCTAATATATATGAATAAAGT

G 3'

5/7

Figure 5

Figure 6

A   bovine serum albumin

B   ovalbumin

C   carbonic anhydrase

D   trypsin inhibitor (soy bean)

E   lysozyme


1:   molecular weight marker

2:   protein (with 2-mercaptoethanol)

3:   protein (without 2-mercaptoethanol)

7/7

Figure 7

relative mobility

A     bovine serum albumin

B     ovalbumin

C     carbonic anhydrase

D     trypsin inhibitor

E     lysozyme

| | DOCUMENTS CONSIDERED TO BE RELEVANT | | EP 83109403.2 |
|---|---|---|---|
| Category | Citation of document with indication, where appropriate, of relevant passages | Relevant to claim | CLASSIFICATION OF THE APPLICATION (Int. Cl. ³) |
| T,X, | EP - A2 - 0 089 676 (TAKEDA CHEMI-CAL INDUSTRIES, LTD) | 1,4,6, 10 | C 12 N 15/00 |
| D,P | * Abstract; claims 1,10,18,19 * | | C 12 P 21/00 |
| | -- | | C 07 C 103/52 |
| P,X | EP - A2 - 0 088 540 (BIOGEN N.V.) | 1,4 | A 61 K 45/02 |
| | * Abstract; claims 19,20,25-27 * | | A 61 K 39/395 |
| | -- | | |
| P,X | EP - A2 - 0 077 670 (GENENTECH, INC.) | 1,4 | |
| | * Abstract; claims 1,22 * | | |
| | -- | | |
| X | EP - A2 - 0 063 482 (MELOY LABORA-TORIES, INC.) | 1 | |
| | * Abstract; claim 39 * | | |
| | -- | | **TECHNICAL FIELDS SEARCHED (Int. Cl. ³)** |
| X | NATURE, vol. 296, no. 5854, March 18, 1982, (New York, USA) | 1,4 | C 12 N |
| | H.K. HOCHKEPPEL et al. "Monoclonal antibody against human IFN-$\gamma$". pages 258-259 | | C 12 P |
| | * Totality * | | A 61 K |
| | -- | | |
| D,X | NATURE, vol. 295, no. 5849, February 11, 1982,(New York, USA) | 1,4,7, 8,9 | |
| | P.W. GRAY et al. "Expression of human immune interferon cDNA in E. coli and monkey cells" pages 503-508 | | |
| | * Fig. 3 * | | |
| | -- | | |

The present search report has been drawn up for all claims

| Place of search | Date of completion of the search | Examiner |
|---|---|---|
| VIENNA | 22-02-1984 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| D,X | NUCLEIC ACIDS RESEARCH, vol. 10, no. 8, April 24, 1982 (IRL. Press. Oxford and Washington DC)<br><br>R. DEVOS et al. "Molecular cloning of human immune interferon cDNA and its expression in eucaryotic cells"<br>pages 2487-2501<br><br>   * Abstract; page 2496, line 7 *<br><br>---- | 1,4,7, 8,9 | |
| | | | TECHNICAL FIELDS SEARCHED (Int. Cl.³) |